# EUROPEAN PATENT SPECIFICATION

(11) **EP 1 081 099 B1**
(45) Date of publication and mention of the grant of the patent: **16.06.2004**
(21) Application number: 00307679.1
(22) Date of filing: 06.09.2000
(51) Int. Cl.: C02F 3/34, B09C 1/10, A62D 3/00

(54) **Bioremediation process using bacterivorous protozoa**
Biologische Sanierung mit bakterivoren Protozoen
Procédé de biotraitement en utilisant bactérivorous protozoaires

(30) Priority: 06.09.1999 GB 9920937; 30.09.1999 GB 9923031
(43) Date of publication of application: 07.03.2001
(73) Proprietor: MARINE BIOTECHNOLOGY INSTITUTE CO., LTD., Bunkyo-ku, Tokyo 113-0033 (JP)
(72) Inventor: Mattison, R.G., c/o Marine Biotech. Inst. Co., Ltd, Kamaishi-shi, Iwate 026-0001 (JP)
(74) Representative: McNally, Roisin

(56) References cited:
- WO-A-92/19373
- FR-A- 2 334 630
- LEE N M ET AL: "Reducing sludge production in aerobic wastewater treatment through manipulation of the ecosystem" WATER RESEARCH,ELSEVIER SCIENCE PUBLISHERS, AMSTERDAM,NL, vol. 30, no. 8, 1 August 1996 (1996-08-01), pages 1781-1790, XP004035306 ISSN: 0043-1354
- PATENT ABSTRACTS OF JAPAN vol. 2000, no. 05, 14 September 2000 (2000-09-14) & JP 2000 051886 A (AGENCY OF IND SCIENCE &TECHNOL;KANKYO ENG CO LTD; NAKAMURA KAZUNOR), 22 February 2000 (2000-02-22)

## Description

### FIELD OF THE INVENTION

The present invention relates to a bioremediation process wherein bacterivorous protozoa belonging to Heteromita are used to increase the efficiency of both *in situ* and bioreactor bioremediation processes involving the biodegradation of organic pollutants including petroleum derived hydrocarbons (oils).

### BACKGROUND OF THE INVENTION

This field has considerable commercial potential and numerous companies have already been set up to exploit the ability of bacteria to 'clean up' oil spills in natural environments such as soils.

Contamination with organic pollutants including petroleum-derived hydrocarbons is of widespread importance in the terrestrial (soils, subsurface and groundwater aquifers), aquatic (rivers and lakes) and marine environments. The toxicity associated with many comparatively soluble petroleum-derived hydrocarbons poses a risk not only to the environment but also to human health. This results from the fact that groundwater aquifers, rivers and lakes supply the bulk of our drinking water. The most toxic.and frequently encountered petroleum-derived hydrocarbons are benzene, toluene, ethylbenzene and xylenes (BTEX). These petroleum-derived hydrocarbons are often lighter than water and so when a spill occurs they percolate from the surface through the soil/sediment matrix and often settle in a film at the groundwater table. This water table is variable in depth, say 3-7 metres.

Bioremediation processes designed to treat contaminated sites typically employ bacteria to utilize the organic pollutant as their main carbon and energy source. These process may be divided into two classes, namely (1) *in situ* and (2) *ex situ*. *In situ* bioremediation involves the direct delivery of bacteria and/or oxygen/oxidants to the affected site. *Ex situ* bioremediation involves physical excavation or removal of the affected material with it's subsequent treatment in bioreactor vessels. *In situ* bioremediation is widely considered to be an environmentally friendly and cost effective approach to the treatment of contamination in groundwater aquifers. This approach relies on the collective ability of bacteria present and/or added to degrade pollutants under optimal conditions which include sufficient hydraulic conductivity to permit adequate flow of soluble pollutants and oxygen/oxidants through the affected area. Numerous naturally occurring bacteria are capable of degrading and detoxifying petroleum-derived hydrocarbons. Of particular importance are members of the genus *Pseudomonas* members of the beta subdivision of Proteobacteria most notably the genus *Azoarcus* and members of the delta subdivision of Proteobacteria which include many sulphate reducing bacteria. These bacteria degrade petroleum-derived hydrocarbons under various redox conditions ranging through aerobic to nitrate reducing and sulphate reducing.

The efficiency of *in situ* bioremediation can be limited in two ways namely, (1) if unrestricted growth of bacteria leads to rapid exhaustion of oxidants and other growth factors and/or (2) if unrestricted growth of bacteria leads to bioclogging with a reduction in hydraulic conductivity. In other words a bioclog resulting from bacterial biomass and/or exopolymeric 'slime' produced by many growing bacteria reduces the permeability of a matrix (e.g. soil/sediment) to water. Both phenomena have been noted in the field. They have also been demonstrated in model systems under laboratory conditions in the absence of bacteria feeding (bacterivorous) protozoa.

Furthermore, bioremediation processes involving the combined use of bacteria and protozoa are known. Both Patent documents FR2334630A and WO9219373A describe the use of both bacteria and protozoa in the process for degrading organic pollutants. Additionally the article by N.M.Lee et al: "Reducing sludge production in aerobic wastewater treatment through manipulation of the ecosystem", Water Research Vol.30, No. 8, pp.1781-1790, 1996, describes another process for the treatment of waste water from pulp and paper industries that exploits the addition of bacteria and protozoa to the waste water. However, none of these documents show the effectiveness of bacterivorous protozoa Heteromita in the presence of bacteria for in situ and ex situ bioremediation.

### SUMMARY OF THE INVENTION

In order to solve the problems of the conventional bioremediation processes, the inventor conducted extensive studies and found the following. That is, when a bacterium utilizing the organic pollutant in combination with a protozoa eating such a bacterium are added to organic pollutant, the bioremediation by the bacteria is unexpectedly improved and accelerated, in spite of the bacterivorous characteristics of the protozoa. Based on such a new finding, the present invention was accomplished.

Accordingly, the invention provides a bioremediation process wherein bacterivorous protozoa belonging to Heteromita are used to increase the efficiency of *in situ* or *ex situ* bioremediation process involving the biodegradation of organic pollutants including petroleum derived hydrocarbons. The bacterivorous protozoa are added with appropriate bacteria to contaminated sites or bioreactors in order to facilitate and enhance bioremediation.

### BRIEF DESCRIPTION OF THE DRAWINGS

Fig. 1 shows the structure of a toluene contaminated groundwater aquifer. Transect (1a) and plan (1b) views of the aquifer are shown along with details of the flow cell (1c).

Fig. 2 shows the growth of bacteria on toluene in the presence and absence of flagellates.
Toluene concentration (2a), numbers of bacteria (2b) and numbers of flagellates (2c) in the presence (○) and absence (●) of flagellates are plotted with respect to time. Sterile biotic controls (■) are also shown.

Fig. 3 shows the rate of toluene consumed by bacteria in the presence (□) and absence (■) of flagellates plotted with respect to time. The rate of toluene consumed by the theoretical total number of bacteria ('bacterial equivalents' corresponding with flagellates, and summed with observed numbers of bacteria) is also shown (■).

### DETAILED DESCRIPTION OF THE INVENTION

This method would add bacterivorous protozoa belonging to Heteromita either alone or in combination with appropriate bacteria to contaminated sites or bioreactor in order to facilitate and enhance bioremediation.

The subject for the bioremediation is organic pollutant, especially petroleum-derived hydrocarbon (e.g., benzene, toluene, ethylbenzene and xylene). The subject may be mixed with other components such as water, sea water, sands, soils, etc.

The bacterium for use in the present invention is not particularly limited as long as the bioremediation is possible. Either one type of bacteria or several types of bacteria in combination can be used. Appropriate bacteria would be those capable of degrading the pollutant in question and would also be an acceptable food source for the bacterivorous protozoa Heteromita. Illustrative non-limiting examples of the bacteria include those belonging to the genera *Pseudomonas, Azoarcus, Sphingomonas*, *Comamonas*, *Rhodococcus* and *Acinetobacter* and sulphate reducing bacteria, specifically, those belonging to *Pseudomonas* and *Azoarcus* and sulphate reducing bacteria (e.g., *Desulfobacter*). Bacteria may be isolated and cultured by the conventional method (see, Atlas, R.M. and Parks, L.C. 1993. Handbook of microbiological media. CRC Press, Boca Raton, FL., USA.). Commercially available bacteria, those available from depositary institution (e.g., ATCC, DSMZ, etc.), and those isolated from the nature may also be used. The amount of bacterium to be used is not particularly limited as long as the bioremediation is possible.

The bacterivorous protozoa for use in the present invention belongs to the genera *Heteromita* specifically, those belonging to the species *Heteromita globosa*. An illustrative example of the strain of the bacterivorous protozoa includes *Heteromita globosa* ATCC 50780 Bacterivorous protozoa may be isolated and cultured by the conventional method (see, Kemp, P.F., Sherr, B.F., Sherr, E.B. and Cole, J.J. 1993. Handbook of methods in aquatic microbial ecology. CRC Press, Boca Raton, FL., USA.). Commercially available bacterivorous protozoa, those available from depositary institution (e.g. ATCC), and those isolated from the nature may also be used. The amount of bacterivorous protozoa to be used is not particularly limited as long as improvement and acceleration of the bioremediation is possible.

The combination of bacterivorous protozoa and bacteria includes the combination of *Pseudomonas fluorescens* (especially, DSM 12877 strain) and *Heteromita globosa* (especially, ATCC 50780 strain).

Preferably, a ratio of one bacterivorous protozoa to between 10¹ and 10⁵ (more preferably from 10³ to 10⁴) bacteria would be added to provide a dynamic control of bacterial growth. This would improve the efficiency of bioremediation process by (1) leading to a reduction in bioclogging with subsequent improvement in hydraulic conductivity in the aquifer, and/or (2) increasing the metabolic rate of individual pollutant degrading bacteria.

The bioremediation process itself may be carried out by the conventional method except that the bacterivorous protozoa is added. It is also possible to utilize the naturally occurring bioremediation.

In laboratory cultures containing bacteria, protozoa and hydrocarbons it was noticed that the presence of protozoa actually speeds up (rather than reducing) the breakdown rate of hydrocarbons by bacteria. The invention therefore relates to the effects of protozoa on increasing the breakdown rate of hydrocarbons by bacteria. It is .therefore proposed that commercial companies could utilize protozoa to improve and accelerate their existing bioremediation processes. Since hydrocarbon spills are of global importance, any additional improvement to bioremediation processes could be very significant.

The mechanism of the improvement and acceleration of the bioremediation according to the present invention is not sufficiently clear but the following mechanism is possible explanation.

Bacterivorous protozoa appear to reduce the phenomenon of bacterial bioclogging and in so doing improve hydraulic conductivity in aquifers.

Bacterivorous protozoa accelerate biodegradation and mineralization of such organic pollutants.

Bacterivorous protozoa increase the bioavailability of mineralized organic pollutants by contributing carbon, nitrogen and phosphorus to the ecosystem.

Comparatively large populations of these bacteria along with bacterivorous protozoa occur at organically contaminated groundwater aquifers and other sites. Large populations of bacteria cir 10⁸/gram of soil/sediment and protozoa 10⁵/gram of soil/sediment occur in the surface and normally decrease with depth. In the presence of the organic pollutants mentioned the numbers of bacteria and protozoa at the water table is comparable with that found at the surface. These protozoa are comprised of ciliates, flagellates and amoebae with flagellates being the most numerous group in groundwater aquifers. Most ciliates are absent below a depth of about 1 metre. The role of bacterivorous protozoa Heteromita in *in situ* bioremediation is considered to be an indirect result of their ability to selectively graze on and control the biomass of the bacterial community. A controlled bacterial biomass would lead to a reduction in bioclogging with subsequent improvement in hydraulic conductivity. Protozoa may also enhance bioremediation by increasing the metabolic rate of pollutant degrading bacteria. They may also facilitate this by remineralising growth limiting nutrients such as ammonium and phosphorus.

Assume that a pristine groundwater aquifer has the following characteristics. It is comprised of a sandy soil with a gravel/clay lens and an underlying base of impervious clay/rock. The groundwater flow rate is 2 metres/day. An indigenous population of bacteria (approx. 10⁷/gram sediment) and protozoa (approx. 10⁴/gram sediment) occurs at the water table. The protozoan community is comprised mostly of bacterivorous flagellates.

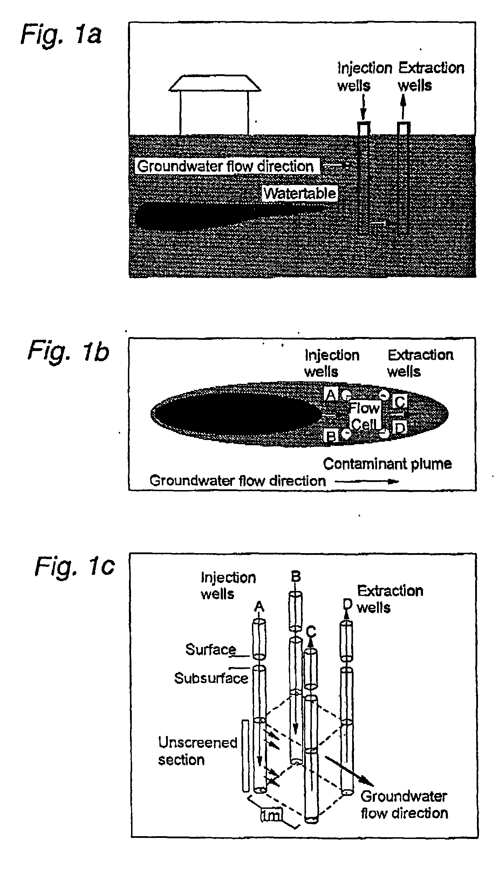

Assume that this aquifer is contaminated by a spill of petroleum derived hydrocarbons comprised mainly of toluene (Fig. 1a). Assume that 10³ litres of toluene forms a film or non aqueous phase liquid (NAPL) in the saturated aquifer below the water table. The indigenous population of bacteria and protozoa at the water table increases to approx. 10⁸/gram sediment and 10⁵/gram sediment, respectively. Only 1% of the indigenous population of bacteria can degrade toluene.

Assume that immediately downstream of the NAPL some four wells are installed in an arrangement shown in Fig. 1b & 1c. These wells are located at the corners of a 1 metre square and extend into the saturated aquifer below the water table. Each well is screened except for the bottom 1 metre in the saturated aquifer which is left unscreened. The unscreened sections of the four wells delimit a volume of 1 cubic metre sediment in the saturated aquifer. This cube (approx. 500 kilograms sediment) will be referred to as the flow cell. Wells A and B are the injection wells while C and D are the extraction wells.

Assume that toluene achieves maximum saturation in the groundwater (5.6 mM) and is transported horizontally in a plume downstream of the NAPL at 2 metres /day through the flow cell. Thus approx. 1 litre /day toluene passes through the flow cell.
(1) Estimation of the consumption of toluene by the indigenous bacterial population in the untreated aquifer
   Assume that toluene is consumed at a rate of approx. 2 micromoles /gram sediment/day. The amount of toluene consumed in the flow cell is approx. 1 mole /day which corresponds with approx. 100 millilitres /day. Therefore in the untreated aquifer approx. 10% of toluene transiting the flow cell is consumed.
(2) Estimation of the consumption of toluene during *in situ* bioremediation without accounting for bioclogging
   Assume that sufficient oxidants, nutrients and toluene degrading bacteria are added to injection.wells A and B to support the population of introduced bacteria at approx. 10⁹ cells/ gram sediment. The introduced population is initially ten fold higher than the indigenous population and would be expected to increase. Assume this results in a minimum ten fold increase in toluene degrading ability. Therefore in the *in situ* bioremediated aquifer approx. 100% of toluene transiting the flow cell is consumed.
(3) Estimation of the consumption of toluene during *in situ* bioremediation accounting for bioclogging
   Assume that progressive bioclogging occurs from the injection wells and results in approx. one hundred fold reduction in hydraulic conductivity across the flow cell after approx. 30 days. Groundwater flow is reduced and the introduced and indigenous population of bacteria in the flow cell become progressively depleted in oxidants, nutrients and toluene. Therefore bioclogging will result in an approx. 99% reduction in toluene transiting the flow cell after one month. Furthermore, in the short term, bioclogging will be a largely irreversible phenomenon.
(4) Estimation of the consumption of toluene during in situ bioremediation with the addition of bacterivorous protozoa

Assume that sufficient bacterivorous protozoa are added to injection wells to support the population of introduced protozoa at approx. 10⁶ cells/gram sediment. The introduced population is ten fold higher than the indigenous population and maintains an equilibrium ratio of approx. 1:1000 with introduced bacteria. Protozoan control on the bacterial population would significantly reduce the development of bioalogging in the flow cell as described in (3). Therefore in the *in situ* bioremediated aquifer, the addition of bacterivorous protozoa should facilitate those conditions favourable to the degradation of toluene as described in (2).

### EXAMPLE I

A model food chain which was developed to investigate the influence of grazing by bacterivorous flagellates on bacteria degrading toluene in batch culture.

The rate of toluene consumed by *Pseudomonas fluorescens* DSM 12877 (max. 0.37 femtomoles/cell/hour) was significantly higher in the presence of the bacterivorous *flagellate Heteromita globosa* ATCC 50780 (max. 1.38 femtomoles/cell/hour). A maximum increase of up to 7.5 times was observed in the rate of toluene consumed by these bacteria during exponential growth of this flagellate.

The *Pseudomonas fluorescens* was isolated using sediment from a diesel contaminated aquifer (depth 4 metres) near Studen, Switzerland. Sediment was enriched on 1.0% diesel and bacterial isolates were grown aerobically on diluted Bactopeptone-trypticase-yeast-glucose agar medium (1:20) at 25°C (see Balkwill, D.L., and Ghiorse, W.C. (1985) Appl. Environ. Microbiol. 50: 580-588). Isolates and replated (x3) for purity then inoculated into liquid culture containing minimal salt medium (see Thomas, J.M., Bruce, C.L., Gordy, V.R., Duston, K.L., Hutchins, S.R., Sinclair, J.L., and Ward; C.H. (1997) J. Indust. Microbiol. Biotechnol. 18:213-221) and 1 mM of either benzene, ethylbenzene, toluene or xylenes (o-, m-, p-) supplied as sole carbon source. Bacterial growth was indicated by measuring an increase in cell turbidity and reduction in concentration of carbon source. An isolate with toluene degrading ability was characterized and deposited with the German Collection of Microorganisms and Cell Cultures (DSMZ) (Braunschweig, Germany; DSM 12877)

The *Pseudomonas fluorescens* was grown on mineral salts medium (1.8 litre) containing toluene (10% v/v) adsorbed in 2,2,4,4,6,8,8,-hepta-methylnonane (20 millilitres/litre). The medium was gently stirred overnight to reach an aqueous equilibrium concentration of 1 mM toluene. Bacteria were added and the culture was stirred and aerated (1 day, 25°C) then harvested during log phase growth. Harvested bacteria were washed by centrifugation (7,500 g x 3) then resuspended in soil extract salts (SES) medium and used immediately. Bacteria were unable to grow in SES medium as a sole carbon source. The SES medium contained (gram/litre) : KH₂PO₄ (0.46), Na₂HPO₄ (3.10), MgSO₄.7H₂O (0.02), KNO₃ (0.2) and soil extract (100 millilitre). Nitrogen and phosphorus were provided in excess and were not growth limiting. The pH was adjusted to 7.0 by adding HCl (1 N) prior to autoclaving. Soil extract was prepared according to Rønn et al. (see, Rønn, R., Ekelund, F. and Christensen, S. (1995) Pedobiologia 39, 10-19).

The *Heteromita globosa* was isolated using sediment from a BTEX contaminated aquifer at an abandoned oil refinery near Hunxe, Germany. Sediment was inoculated into various liquid media (including SES medium) and microcosms were maintained at 25°C and examined after 14 days. Growth of flagellates was determined by counting in a Neubauer chamber. Live bacteria were stained with the fluorescent dye 4,6 Diamidino-2-phenylindole (1 microgram/millilitre; 10 minutes) and washed (x3) by centrifugation in minimal salt medium (3000 grams; 10 minutes). Stained bacteria were fed to these flagellates then examined microscopically for evidence of internalization and digestion.

Growing flagellates were reinoculated into fresh medium with *P*. *fluorescens* and later harvested. A two fold dilution series of flagellates in SES medium was prepared to their extinction point in multiwell plates. The highest dilution showing growth was used to establish clonal cultures. One culture was deposited with the American Type Culture Collection (ATCC) (Manassas, VA, USA; ATCC 50780)

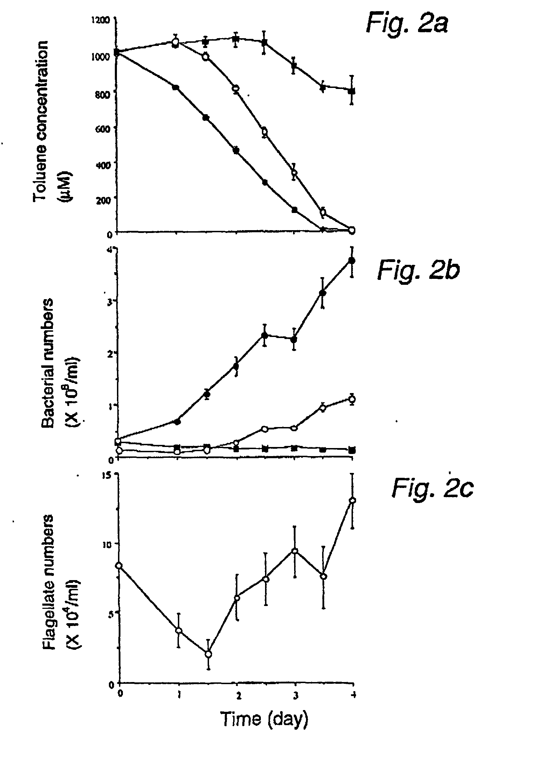

Culture bottles (250 millilitres) equipped with teflon®™ seals were divided into three experimental groups consisted of two treatments and one biotic control with six replicates in each. Treatments were inoculated with bacteria (1x10⁸ cells/millilitre) in SES medium and divided according to the absence or presence of flagellates (1x10⁵ cells/millilitre). Biotic controls were inoculated with both bacteria and flagellates in SES medium and were sterilized by autoclaved (121°C for 20 minutes). Toluene was added to a final concentration of approx. 1 mM and bottles were incubated at 25°C without. shaking. Samples were removed from each bottle for measurement of cell numbers and toluene concentration at regular intervals until 6 day. Data are-shown in Fig. 2.

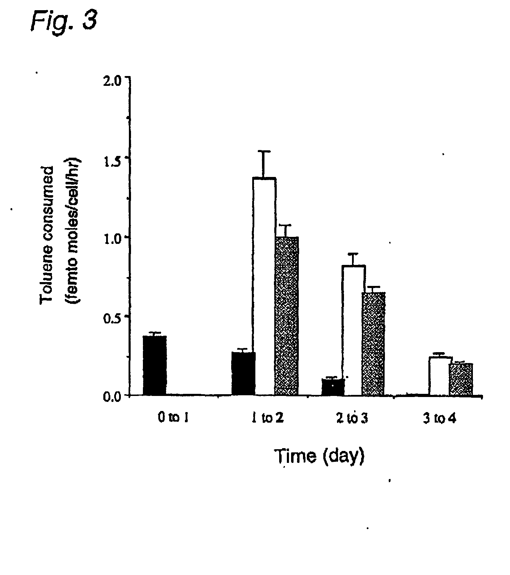

Rates of toluene consumed by bacteria at different growth stages and in the presence or absence of flagellates were calculated from changes in mean toluene concentration (corrected for toluene sorption in biotic controls) and mean numbers of bacteria measured over daily time increments up to 6 day. Numbers of flagellates were converted into their 'bacterial equivalents' based on the number of:bacteria required to produce one flagellate division. These 'bacterial equivalents' were then summed with observed numbers of bacteria to provide a theoretical estimate of the total numbers of bacteria assuming no flagellate grazing had occurred. (Fig. 3)

No significant differences (p=0.729) were observed between rates of toluene consumed by bacteria in the presence of flagellates when calculated with or without inclusion of 'bacterial equivalents'. This suggested the effect was not due simply to differences in bacterial numbers but could result from an increase in specific metabolic activity of bacteria presumably induced by the presence of flagellates.

## Claims

1. A bioremediation process comprising adding at least one bacterium which is capable of utilising an organic pollutant and at least one bacterivorous protozoa to the organic pollutant, wherein the bacterivorous protozoa belongs to *Heteromita*.

2. The process as claimed in Claim 1 wherein the bacterium is a member belonging to the genus *Azoarcus*.

3. The process as claimed in Claim 1 and Claim 2, wherein the bacterium is a sulphate reducing bacteria.

4. The process as claimed in Claim 1, wherein said bacterium is *Pseudomonas fluorescens* and said protozoa is *Heteromita globosa*.

5. The bioremediation process as claimed in any one of the preceding Claims, wherein the ratio of the bacterivorous protozoa to bacterium is from about 1-10 to about 1-10⁹.

6. The bioremediation process as claimed in any one of the preceding Claims, wherein said organic pollutant is a petroleum derived hydrocarbon.

7. The bioremediation process as claimed in Claim 6, wherein said petroleum derived hydrocarbon is selected from the group consisting of benzene, toluene, ethylbenzene and xylene.

8. A method for increasing efficiency of an in situ bioremediation process, which comprises adding a bacterivorous protozoa to the process, wherein the bacterivorous protozoa belongs to Heteromita.

9. A method for increasing efficiency of a bioremediation process in batch culture or bioreactor, which comprises adding a bacterivorous protozoa to the process, wherein the bacterivorous protozoa belongs to *Heteromita*.

## Patentansprüche

1. Ein Bioreinigungsvorgang, der das Hinzugeben von mindestens einer Bakterie, die einen organischen Schadstoff nutzen kann, und mindestens einem bakerivoren Protozoon zu dem organischen Schadstoff beinhaltet, wobei das bakterivore Protozoon *Heteromita* angehört.

2. Vorgang gemäß Anspruch 1, wobei die Bakterie der Gattung *Azoarcus* angehört.

3. Vorgang gemäß Anspruch 1 und Anspruch 2, wobei die Bakterie eine Sulfat reduzierende Bakterie ist.

4. Vorgang gemäß Anspruch 1, wobei die Bakterie *Pseudomonas fluorescens* und das Protozoon *Heteromita globosa* ist.

5. Bioreinigungsvorgang gemäß einem der vorhergehenden Ansprüche, wobei das Verhältnis des bakterivoren Protozoons zu der Bakterie zwischen ungefähr 1-10 und ungefähr 1-10⁹ liegt.

6. Bioreinigungsvorgang gemäß einem der vorhergehenden Ansprüche, wobei der organische Schadstoff ein aus Erdöl erzeugter Kohlenwasserstoff ist.

7. Bioreinigungsvorgang gemäß Anspruch 6, wobei der aus Erdöl erzeugte Kohlenwasserstoff aus der Gruppe ausgewählt ist, die aus Benzen, Toluen, Ethylbenzen und Xylen besteht.

8. Ein Verfahren zur Erhöhung der Wirksamkeit eines In-situ-Bioreinigungsvorgangs, das das Hinzugeben eines bakterivoren Protozoons zu dem Vorgang beinhaltet, wobei das bakterivore Protozoon *Heteromita* angehört.

9. Verfahren zur Erhöhung der Wirksamkeit eines Bioreinigungsvorgangs in diskontinuierlicher Kultur oder in einem Bioreaktor, das das Hinzugeben eines bakterivoren Protozoons zu dem Vorgang beinhaltet, wobei das bakterivore Protozoon *Heteromita* angehört.

## Revendications

1. Un processus de bioremédiation comportant l'ajout d'au moins une bactérie qui est capable d'utiliser un polluant organique et d'au moins un protozoaire bactérivore au polluant organique, dans lequel le protozoaire bactérivore appartient aux *Heteromita.*

2. Le processus tel que revendiqué dans la revendication 1 dans lequel la bactérie est un élément appartenant au genre *Azoarcus.*

3. Le processus tel que revendiqué dans la revendication 1 et la revendication 2, dans lequel la bactérie est une bactérie réductrice de sulfate.

4. Le processus tel que revendiqué dans la revendication 1, dans lequel ladite bactérie est *Pseudomonas fluorescens* et ledit protozoaire est *Heteromita globosa*.

5. Le processus de bioremédiation tel que revendiqué dans une quelconque des revendications précédentes, dans lequel les proportions relatives de protozoaire bactérivore et de bactérie vont d'environ 1/10 à environ 1/10⁹.

6. Le processus de bioremédiation tel que revendiqué dans une quelconque des revendications précédentes, dans lequel ledit polluant organique est un hydrocarbure dérivé du pétrole.

7. Le processus de bioremédiation tel que revendiqué dans la revendication 6, dans lequel ledit hydrocarbure dérivé du pétrole est sélectionné dans le groupe consistant en benzène, toluène, éthylbenzène et xylène.

8. Une méthode destinée à accroître l'efficacité d'un processus de bioremédiation in situ, laquelle comporte l'ajout d'un protozoaire bactérivore au processus, dans laquelle le protozoaire bactérivore appartient aux *Heteromita*.

9. Une méthode destinée à accroître l'efficacité d'un processus de bioremédiation en culture en série ou en bioréacteur, laquelle comporte l'ajout d'un protozoaire bactérivore au processus, dans laquelle le protozoaire bactérivore appartient aux *Heteromita*.
